# EUROPEAN PATENT SPECIFICATION

(11) **EP 1 655 032 B1**
(45) Date of publication and mention of the grant of the patent: **07.10.2009**
(21) Application number: 04766900.7
(22) Date of filing: 04.08.2004
(51) Int. Cl.: A61K 31/675, A61K 31/185, A61K 31/53, A61P 25/28

(54) **USE OF THE P2X7 ANTAGONISTS o-ATP OR BBG FOR THE TREATMENT OF THE NEURODEGENERATIVE PHASE OF MULTIPLE SCLEROSIS**
VERWENDUNG DER P2X7 ANTAGONISTEN o-ATP oder BBG ZUR BEHANDLUNG VON DER NEURODEGERATIVE PHASES DES MULTIPLE SKLEROSIS
UTILISATION DES ANTAGONISTES P2X7 o-ATP OU BBG POUR LE TRAITEMENT DE LA PHASE NEURDEGERATIVE DE LA SCLÉROSE EN PLAQUES

(30) Priority: 04.08.2003 ES 200301853
(43) Date of publication of application: 10.05.2006
(73) Proprietor: UNIVERSIDAD DEL PAIS VASCO-EUSKAL HERRIKO UNIBERSITATEA, 48940 Leioa (ES)
(72) Inventor: MATUTE ALMAU, Carlos, E-48940 LEIOA (Bizkaia) (ES); ALBERDI ALFONSO, Elena, E-48990 GETXO (Vizcaya) (ES); DOMERCQ GARCIA, Maria, E-48014 BILBAO (ES); PEREZ SAMARTIN, Alberto, E-48600 SOPELANA (Vizcaya) (ES); PEREZ CERDA, Fernando, E-48014 BILBAO (ES); TORRE MARTINEZ, Iratxe, E-01470 AMURRIO (Alava) (ES); SANCHEZ GOMEZ, Maria, Victoria, E-48940 GETXO (Vizcaya) (ES)
(74) Representative: Carpintero Lopez, Francisco
(86) International application number: PCT/ES2004/000361
(87) International publication number: WO 2005/014007

(56) References cited:
- WO-A2-98/03178
- WO-A2-99/38532
- WO-A2-03/103675
- ZIYAL R. ET AL.: 'Vasoconstrictor responses via P2X-receptors are selectively antagonized by NF023 in rabbit isolated aorta and saphenous artery' BRITISH JOURNAL OF PHARMACOLOGY vol. 120, no. 5, 1997, pages 954 - 960, XP002994899
- KIMURA H. ET AL.: 'Suramin-induced reversal of chronic cerebral vasospasm in experimental subarachnoid hemorrhage' J. NEUROSURG. vol. 97, 2002, pages 129 - 135, XP002996216
- HONORE P. ET AL.: 'TNP-ATP, a potent P2X3 receptor antagonist, blocks acetic acid-induced abdominal constriction in mice: comparison with reference analgesics' PAIN vol. 96, no. 1-2, March 2002, pages 99 - 105, XP002996206
- FUKUHARA N. ET AL.: 'Regulation of the development of allodynia by intrathecally administrated P2 purinoceptor agonists and antagonists in mice' NEUROSCIENCE LETTERS vol. 292, no. 1, 29 September 2000, pages 25 - 28, XP002996207

## Description

### FIELD OF THE INVENTION

The present invention is related to the field of multiple sclerosis, as well as with the use of antagonist substances of the P2X7 receptors, present in oligodendrocytes, for the treatment of the aforementioned diseases, and with compositions which may contain the aforementioned antagonists.

### BACKGROUND TO THE INVENTION

Multiple sclerosis (MS) is the most common demyelinating disease of the central nervous system. It affects one and a half million people in the world, and its symptoms generally appear in young adults, therefore its consequences at a personal and socioeconomical level are very serious.

It is thought that susceptibility to MS is due to unknown genetic and environmental factors. The prevalence of the disease is between 50 to 100 persons per 100,000 inhabitants in regions of high risk, which are mainly located in the northern part of the northern hemisphere, in Europe and America. The risk of suffering from MS increases 10-20 fold in first degree relatives of patients, and concordance between monozygotic twins (genetically identical) is increased by 30%-50%, while in dizygotic twins it only rises to 2%-5%. The genetic susceptibility is not characterised. Up to this moment there is evidence that it can reside in some polymorphism of the genes which code human leucocyte antigens (HLA), myelin oligodendrocyte glycoprotein (MOG) and other genes of chromosomes 10 and 15.

There is a consensus among investigators of MS that it has two phases, an initial inflammatory one, autoimmune in character, and another second one, progressive neurogenerative. In the first, activated T cells cross the blood-brain barrier, and once within the Central Nervous System they liberate pro-inflammatory cytokines which trigger an immunological cascade which ends in the destruction of myelin and death of the oligodendrocytes. The knowledge with a certain detail of the autoimmune process has come in useful for developing agents of an immunomodulatory character whose therapeutic efficacies are very modest. However, no medication has been created which delays or stops the advance of the neurodegenerative phase of the disease which follows a course of progressive neurological deterioration and disability, and which is characterised by the appearance of severe demyelinating lesions in the white matter with a massive loss of oligodendrocytes, atrophy and severe axonal damage. Up until now, different targets for intervention during the inflammatory phase of multiple sclerosis have been described (Zamvil and Steinman, 2003, Neuron 38, 685-688). Among them are found those which are directed to reducing the inflammation of the nervous system started by the activation of myelin specific T cells, which promote autoimmunity particularly against components of myelin, penetrate the central nervous tissue and are released in the pro-inflammatory cytokines such as γ -interferon and tumour necrosis factor- α. β- interferon immunomodulator, approved for the treatment of remittent-recurrent multiple sclerosis, also prevents cellular interactions which lead to penetration of the activated T cells through the vascular endothelium. Other treatments in clinical trial phase are directed towards neutralising the activity of the pro-inflammatory cytokines and/or boost the anti-inflammatories ones. A recent study (Youssef et al., 2002, Nature 420, 78-84) has demonstrated that the drug atorvastatin, used in the treatment of hypercholesterolaemia, is also a potent immunomodulator which prevents or reverses chronic EAE by means of increasing the secretion of anti-inflammatory cytokines and the inhibition of the production of pro-inflammatory cytokines. Purinergic receptors are a type of membrane receptor activated by extracellular purines such as ADP and ATP and which mediate different biological effects, such as the modulation of neuronal activity, the release of neurotransmitters, glycogenolysis, vessel wall contractility or certain immunological processes, etc. The purinergic receptors are classified into two large groups called P1, whose activation is mediated by adenosine, and P2 whose endogenous ligands are ATP and ADP purines and the UTP and UDP pyrimidines. The P1 receptors transduce the signal to the interior of the cell through G- proteins and depending on their molecular, biochemical or pharmacological are subdivided into four groups: A1, A2A, A2B and A3. For their part, the P2 are divided into ionotropic (P2X) and metabotropic (P2Y) (Barnard et al, 1997; Ralevic and Burnstock, 1998).

In recent years it has been demonstrated that the purinergic receptors, besides participating in signals common to neurotransmission, also mediate effects on the glial cells (Rathbone et al, 1999). In fact, the expression of the purinergic receptors in the central nervous system is not only limited to neurons, but also affect the glia (Dunn et al, 2001; Franke et al, 2001 a; Stevens et al, 2002). In particular, purinergic signalling in the astrocytes and microglia act as a means of glia-glia and glia-neuron communication (Fields and Stevens, 2000). Also, some very recent studies indicate the presence of functional receptors in oligodendrocytes *in vitro* (Stevens et al, 2002), which point to a relevant participation in functions common to this cell type. In particular, Stevens et al (2002) show that the adenosine released from the axons due to electrical activity, inhibit the proliferation of oligodendroglial precursors, stimulate their differentiation and promote the formation of myelin.

The signalling by purinergic receptors is also important in cell viability in response to cerebral pathological processes (reviewed in Abbracchio and Burnstock, 1998). Thus, they are involved in the gliotic response to nerve damage (Franke et al, 2001b; James and Butt, 2001), and in the repair response of the central nervous system by means of the production of trophic factors in astrocytes (Ciccarelli et al, 2001). For its part, the presence of ectonucleotidases which break down ATP to adenosine is a neuroprotector element in ischaemia (Braun et al, 1998), while ATP causes glial cell death (Honda and Kohsaka, 2001).

Knowledge on the involvement of the purinergic system in multiple sclerosis is very limited. That information indicates that there are alterations in the activity of 5'-nucleotidase, the enzyme which breaks down ATP to adenosine. This activity is higher in the blood monocytes cultivated for several days in multiple sclerosis patients (Armstrong et al, 1988). For their part, the regions of the central nervous where lesions common to multiple sclerosis are produced have a lower nucleotidase activity (Ansari et al, 1978), which can produce higher concentrations of extracellular ATP and an increased activation of the P2 purinergic receptors.

The novelty of the present invention is based on the discovery on the part of the inventors in that the administration of a determined quantity of some P2X7 receptor antagonists, selected from BBG or oxidised ATP (hereinafter o-ATP), a selective inhibitor of the P2X7 receptors, causes a remission in the symptoms of the disease.

### SUMMARY OF THE INVENTION

The problem to resolve by the present invention is to provide a series of compounds for the treatment of neurodegenerative phase of multiple sclerosis.

The solution presented in this document is based on the capacity possessed by the P2X7 purinergic receptors to stop the development of the aforementioned disease in *vivo* as well as *in vitro* studies.
The invention is illustrated in the example where the studies carried out by the inventors are described in which on the one hand it is demonstrated that the oligodendrocytes in cultures express P2X7 receptors on their surface and, on the other, that the activation of the same with ATP produces an increase in cytosolic calcium and, if the stimulation is prolonged, it finally causes cell death. Likewise, studies are described in which it is demonstrated in *in vivo* and *in vitro* models of multiple sclerosis, that treatment with antagonists to P2X7 purinergic receptors slow down the development of the disease.
Therefore, one aspect of the invention refers to the use of P2X7 receptor antagonists, selected from BBG or o-ATP for the treatment of neurodegenerative phase of multiple sclerosi.s

### BRIEF DESCRIPTION OF THE FIGURES

**Figure 1** shows the electrophysiological properties of the P2X receptors in cultured oligodendrocytes. The activation of the aforementioned receptors produces an input current which can be increased in the absence of divalent ions. The dose-response curves of the natural endogenous antigen, ATP, and its analogues, such as BzATP, indicate that the properties of the response are similar to those of recombinant P2X7 receptors expressed in heterologous systems.
**Figure 2** demonstrates that, both ATP and BzATP produce an elevated increase in the concentration of intracellular calcium which is prevented in the presence of PPADS, a wide spectrum P2X and P2Y antagonist, and also by eliminating calcium from the extracellular medium. Also, it is seen that the responses are increased with propofol and are inhibited with o-ATP, a selective antagonist of the P2X7 receptors.
**Figure 3** demonstrates that the application of ATP or BzATP for 15 minutes causes the death of the oligodendrocytes in culture. Death is calcium dependent, since its elimination from the culture media leads to it not being caused. The wide spectrum antagonist, PPADS is capable of preventing it, if it is applied at the same time as the agonists.

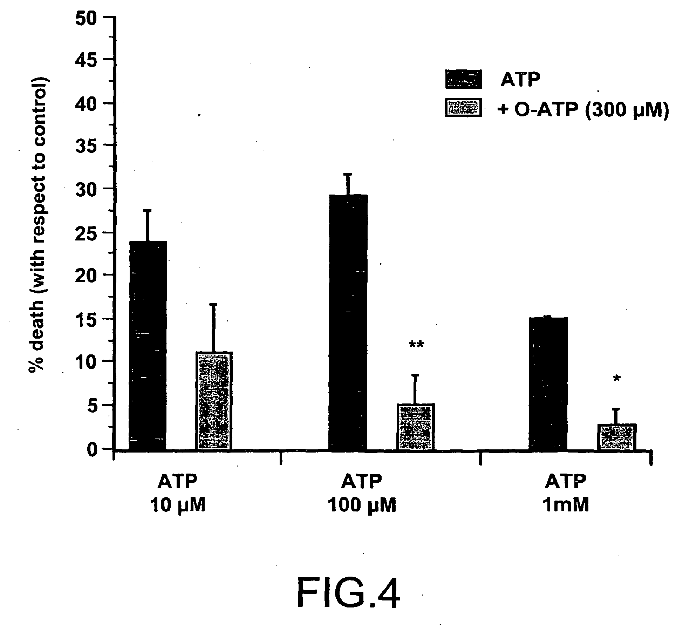

**Figure 4** demonstrates that oligodendroglial death due to ATP can be prevented by means of the selective antagonist of P2X7, o-ATP.
**Figure 5** shows the expression *in situ* of P2X receptors in oligodendrocytes of the optic nerve by means of immunohistochemical techniques using specific antibodies. It is observed that the P2X2, P2X4 and P2X7 receptors (green) are very abundant in oligodendrocytes (red) of the optic nerve. The yellow colour indicates the overlap of both colours, therefore the aforementioned receptors are abundantly expressed in oligodendrocytes. In the same way, it is clear that these are not expressed very much in astrocytes.
**Figure 6** shows how the slow infusion (1 µl/hour) of BzATP (100 mM) produces lesions in the optic nerve, in which tissue damage can be seen with astrogliosis and microgliosis, as well as the disappearance of myelin in the damaged area and breaking up of the axons.
**Figure 7** demonstrates that rats in which EAE is induced have severe neurological symptoms which include paralysis of the limbs and even death. However, treatment with o-ATP before the appearance of symptoms causes the virtual disappearance of the symptoms.
**Figure 8** demonstrates how, twelve days after the induction of EAE, the administration of o-ATP makes the neurological symptoms caused by the disease disappear.
**Figure 9** demonstrates that in EAE the levels of the P2X2 receptors do not change significantly, however, those of P2X7 drop drastically. This indicates that there is a loss of cells which express it, mainly oligodendrocytes.

### DETAILED DESCRIPTION OF THE INVENTION

The first aspect of the invention refers to the use of P2X7 purinergic receptor antagonists for the treatment of neurodegenerative phase of multiple sclerosis. Autoimmunity requires the activation of a precise cascade of processes in cells of the immune system. One part of these cells, the macrophages and the lymphocytes, express P2X1, P2X2, P2X5 and P2X7 receptors, and the activation of the latter causes the release of pro-inflammatory cytokines such as tumour necrosis factor- α(TNF-α) and IL-1β as well as apoptosis by mechanisms which are still not characterised (Burnstock, 2002, Arteriorscler Thromb Vasc Biol. 22, 364-373). However, the exact functions which mediate the P2X receptors in the immune system are still not well understood. It is this expression of P2X receptors in cells of the immune system which makes the use of the P2X receptor antagonists suitable for the treatment of autoimmune diseases.

Among the P2X receptor antagonists there are some which are called wide spectrum owing to the fact that they are capable of binding themselves to several of the family of P2X receptors, although with different affinities to each one of them; and others which are selective to a group of receptors from the P2X family.

The following formulas represent some of these wide spectrum P2X receptor antagonists. Only compounds of formula VII and IX are part of this invention.

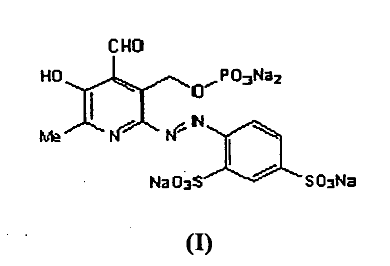

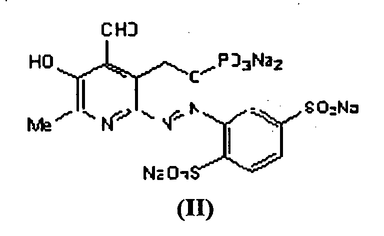

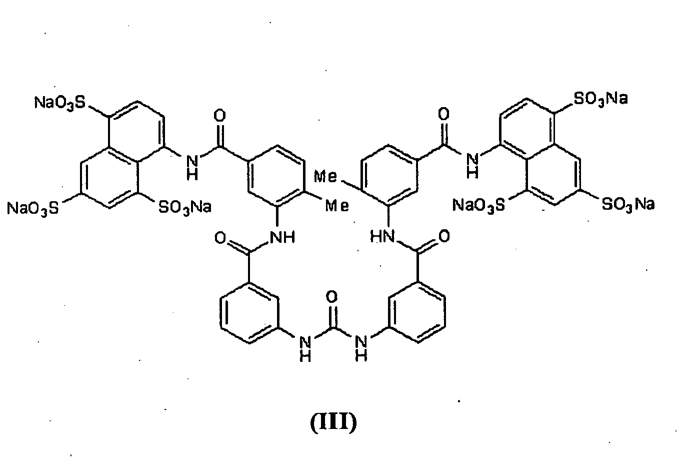

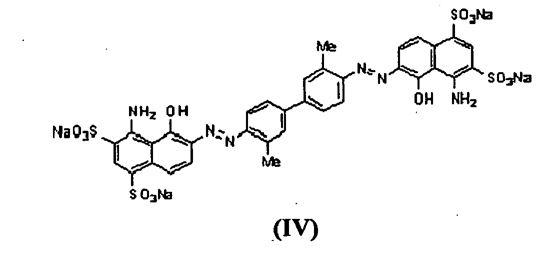

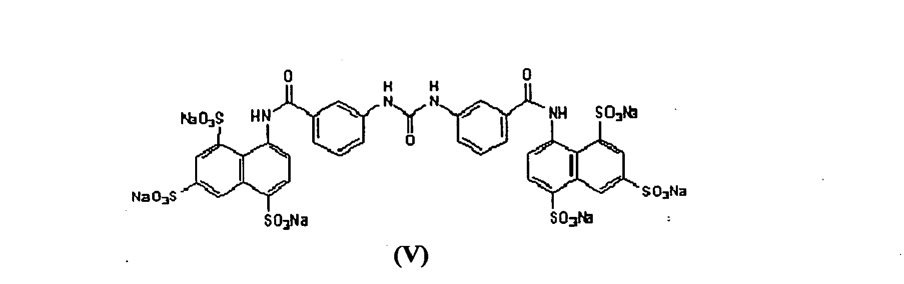

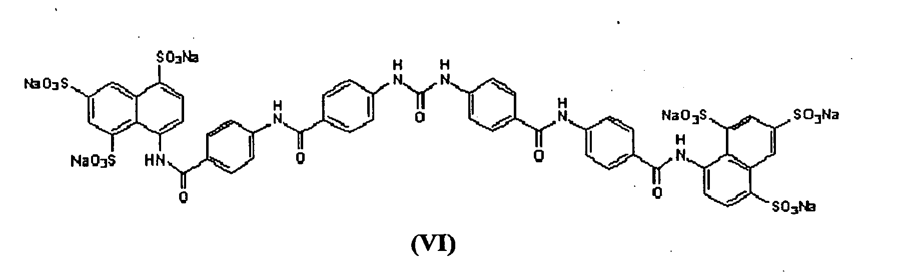

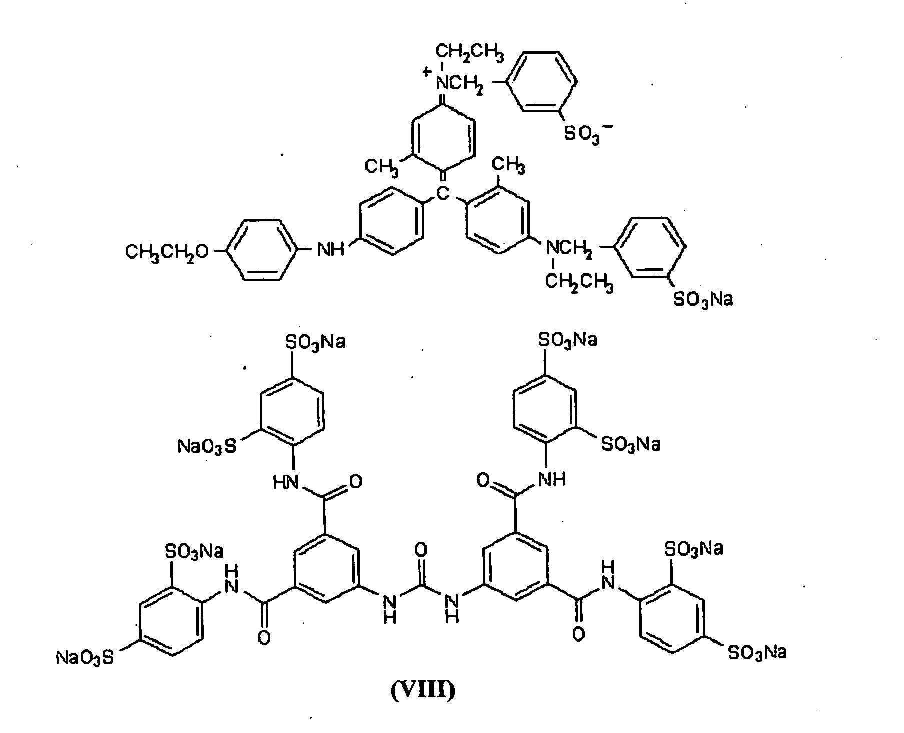

The following formulas represent the selective P2X receptor antagonists:

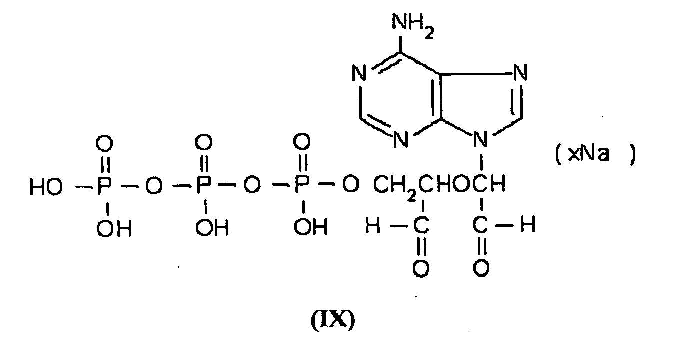

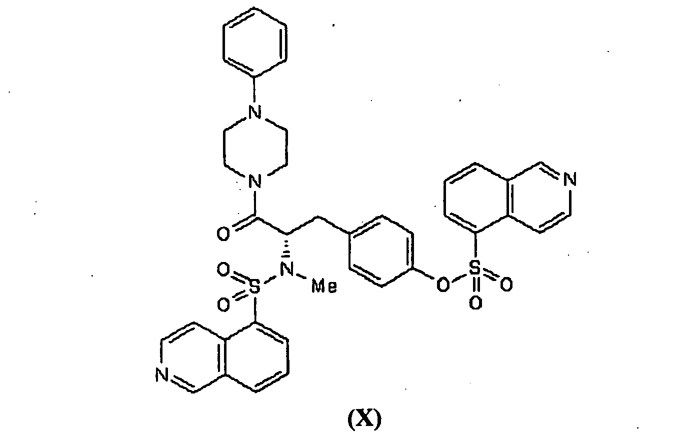

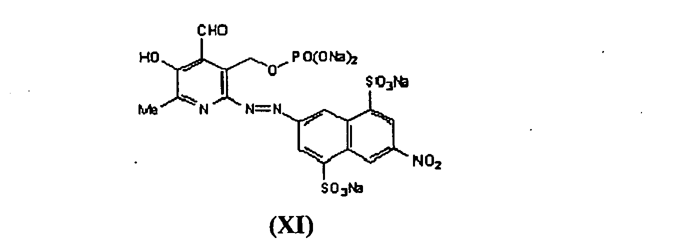

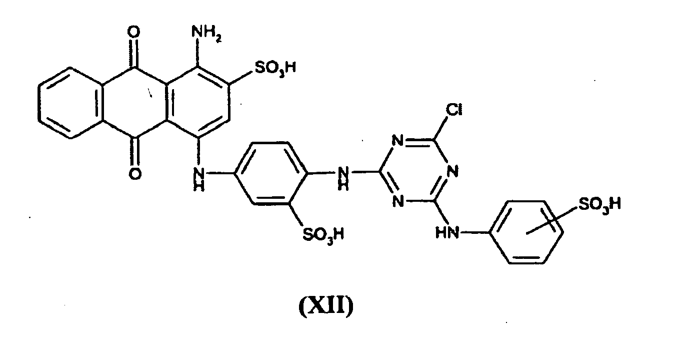

The compounds previously represented by their structural formulas are:
■ **PPADS** (tetrasodium salt of pyridoxalphosphate-6-azophenyl-2',4'-disulfonic acid) **(I)**
■ **iso-PPADS** (tetrasodium salt of pyridoxalphosphate-6-azophenyl-1',4'-disulfonic acid) **(II)**
■ **Suramin** (hexasodium salt of 8,8'-[carbonylbis[imino-3,1-phenylenecarbonylimino(4-methyl-3,1-phenylene)carbonylimino]]bisnaphthalene-1,3,5-trisulphonic acid **(III)**
■ **Evans Blue** (tetrasodium salt of 6,6'-[(3,3'-Dimethyl[1,1'-biphenyl]-4,4'-diyl)bis[4-amino-5-hydroxy-1,3-naphthalenedisulfonic acid]) **(IV)**
■ **NF023** (hexasodium salt of 8,8'-[carbonylbis[imino-3,1-phenylenecarbonylimino]bis-1,3,5-naphthalenetrisulfonic acid] **(V)**
■ **NF279** Hexasodium salt of (8,8'-[carbonylbis(imino-4,1-phenylenecarbonylimino-4,1-phenylenecarbonylimino))bis(1,3,5-naphthalenetrisulfonic acid] **(VI)**
■ **CBB-G** (Coomassie brilliant blue G)**(VII)**
■ **NF449** (octasodium salt of 4,4',4",4"'-(carbonylbis(imino-5,1,3-benzenetriylbis(carbonylimino)))tetrakis-benzene-1,3-disulfonic acid) **(VIII)**
■ **o-ATP** (sodium salt of Adenosine 5-triphosphate, oxidised with periodate) **(IX)**
■ **KN-62** (ester of 4-[(2*S*)-2-[(5-isoquinolinylsulfonyl)methylamino]-3-oxo-3-(4-phenyl-1-piperazinyl)propyl] phenyl isoquinolinesulfonic acid) **(X)**
■ **PPNDS** (tetrasodium salt of pyridoxal-5'-phosphate-6-(2'-naphthylazo-6'-nitro-4',8'-disulfonate) (**XI**)
■ **RB2** 1-Amino-4-[[4-[[4-chloro-6-[[3 (or 4)-sulfophenyl]amino]-1,3,5-triazin-2-yl]amino]-3-sulfophenyl]amino]-9,10-dihydro-9,10-dioxo-2-anthracenesulfonic acid) **(XII)**

Besides those mentioned previously there are other wide spectrum antagonists such as **MRS2220** (cyclic pyridoxine-α4,5-monophosphate-6-azo-phenyl-2',5'disulfonate), **Ip51** (pentapotassium salt of P¹,P⁵-Diinosine-5-pentaphosphate) o **TNP-ATP** (monolithium salt of 2',3'-O-2,4,6-trinitrophenyladenosine 5'-triphosphate), as well as selective ones such as, for example, **HMA** (5-(N,N hexamethylene) amiloride).

The IC₅₀ of some of the previous compounds in relation to the different PX2 receptor subgroups are set out in Table 1.

**Table 1- IC₅₀ of P2X antagonists in relation to each P2X receptor subtype**

| **P2X subtypes** | **P2X1** | **P2X2** | **P2X3** | **P2X4** | **P2X5** | **P2X6** | **P2X7** |
|---|---|---|---|---|---|---|---|
| | | | | | | | |
| **Antagonists: IC50 (**µ**M)** | **PPADS:** 1-5 | **PPADS:** 2 | **PPADS: 1** | **PPADS:** 27.5 | **PPADS:** 2.6 | **PPADS:** >100 | **PPADS:** 4.2 |
| | **Suramin:** 1- 5 | **Suramin:** 1-5 | **Suramin:** 3 | **Suramin:** 178 | **Suramin:** 4 | **Suramin:** >100 | **Suramin:** 4 |
| | **NF023:** 0.21 | **NF 023:** 63 | **NF 023:** 29 | **NF 023:** >100 | | | |
| | **NF279:** 002 | **NF279:** 0.77 | **NF279:** 1.6 | **NF279:** >30 | | | **NF279:** 2.8 |
| | | | | | | | **KN-62:** 0.015 |
| | **Evans Blue:** 1-400 | **Evans Blue:** 1-400 | **Evans Blue:** 1-400 | **Evans Blue:** 1-400 | **Evans Blue:** 1-400 | **Evans Blue:** 1-400 | **Evans Blue:** 1-400 |
| | **isoPPADS:** 1-5 | | **isoPPADS:** 1 | | | | |
| | | **RB-2:** 1 | | | | | |
| | | | | | | | **HMA:** 4,5 |
| | | | | | | | **o-ATP** 5 |
| | | | | **BBG:** >10 | | | **BBG:** 0.01 |
| | **Ip5I:** 0.003 | | **Ip5I:** 3 | | | | |
| | **MRS2220:** 10 | | **MRS2220:** 58 | | | | |
| | **NF449:** 0.01 | | **NF449:** <0.006 | | | | |
| | **PPNDS:** 0.015 | | | | | | |
| | **TNP- ATP:** 0.001 | **TNP-ATP:** 1 | **TNP- ATP:** 0.001 | **TNP-ATP:** 15 | | | **TNP-ATP:** >30 |

In a preferred embodiment, one of the aforementioned antagonists is a P2X7 selective receptor antagonist, o-ATP. In studies carried out by the inventors (see example further on) this compound has been shown to be specially suitable for the treatment of the neurodegenerative phase of multiple sclerosis owing to the relative importance of the presence of P2X7 receptors in oligodendrocytes compared to the other P2X receptors.

The preferred wide spectrum inhibitor is BBG. A preferred realisation contemplates a pharmaceutical composition which contains at least o-ATP, a selective antagonist of P2X7 receptors.

In the following example the studies carried out by the inventors are detailed which illustrates the basis of the invention.

### EXAMPLE

### I- EXPERIMENTAL PROCEDURES

### Oligodendrocyte cultures

The cell cultures were carried out from the optic nerve of the perinatal rat (P12) following established protocols, which were adapted and introduced into the laboratory according to a recent description (Matute et al, 1997, Proc. Natl. Acad. Sci. USA 94, 8830-8835).

### Electrophysiological recordings in oligodendrocytes in vitro

The electrophysiological recordings were carried out in 2 to 5 day cultures, and according to the guidelines indicated in previous works (Patneau et al 1994, Neuron12: 357-371). The cells were recorded in a chamber which allowed the composition of the extracellular medium to vary by means of a constant flow (0.5-1 mL/min). The recording electrodes were glass capillaries which contained specific solutions compatible with the cytoplasm ion concentrations. The study of the responses mediated by the purinergic receptors was carried out using the "whole-cell patch-clamp" technique, measuring the currents generated by the external application of selective agonists and antagonists of the aforementioned receptors.

### Measurement of the cytosol levels of calcium in oligodendrocyte cultures

The concentration of cytosol calcium was determined by the method of Grynkiewikcz et al (1985; J. Biol. Chem. 260, 3440-3450). The oligodendrocytes were loaded with 5 mM of Fura-2/AM, and they were then washed and studied in a Zeiss inverted microscope equipped with a monochromator, 40X immersion objective, an Orca high resolution digital camera, and AquaCosmos software (Hamamatsu Photonics). The changes in cytosol calcium levels in response to agonists and antagonists, in the presence and absence of extracellular calcium, were studied. The calibration was performed at the end of the studies by means of the successive application of ionomycin and EGTA, and the calcium concentration was estimated using the measurement of the 340/380 nm ratio.

### Experiments on the isolated optic nerve

The nerves were isolated from young adult rats, and were perfused for 30 min in artificial cerebrospinal fluid (aCSF) saturated in oxygen by bubbling with 95% oxygen and 5% CO2, under conditions comparable to those described for oligodendrocytes in culture (Fern and Möller, 2000, J. Neurosci. 20: 34-42). Next, they were incubated with purinergic agonists and antagonists for different times. Later, the nerves were perfused for 1 to 24 hours with normal aCSF saturated with oxygen. After this time passed the damage was evaluated histologically as we have described *in vivo* (Matute, 1998, Proc. Natl. Acad. Sci. USA 95: 10229-10234), and the biochemical changes which are underlying to this damage were analysed.

### Immunochemical methods in oligodendrocyte cultures, optic nerve and nerve tissue of experimental animals

Commercial antibodies were used for the study of the presence of oligodendroglial lineage markers, components of myelin, astrocytes and microglia. The techniques included immunocytochemistry, immunohistochemistry and immunoblotting (Western blot), all these are described in detail (see for example, Domercq et al, 1999, Eur. J. Neurosci. 11, 2226-2236)

### Application of substances in the optic nerve in vivo

The experiments on the optic nerve were carried out in rabbits (New Zealand White) which, due to their size, enable better manipulation in experimental surgery. The procedure used was that described previously (Matute, 1998, Proc. Natl. Acad. Sci. 95, 10229-10234). The purinergic agonists were applied using osmotic micropumps which released small quantities of solute for a determined time. Later, the effect of this application on the nerve was evaluated by means of a panel of oligodendrocyte markers and their progenitors, myelin, axonal integrity, astrogliosis and microgliosis.

### Induction of experimental autoimmune encephalomyelitis (EAE)

Lewis rats were used which were immunised subcutaneously with basic myelin protein in the back paws (100 micrograms/animal in 100 microlitres) and Freund's adjuvant with 5.5 mg/ml of tuberculosis Mycobacterium H37Ra. The spinal cord was extracted when the animals had symptoms of the disease (12-14 days post-immunisation) and the expression of the purinergic receptors was analysed using immunochemical techniques (immunoblotting and inmunohistochemical).

### II- RESULTS

### Characteristics of the currents mediated by the P2X receptors in oligodendrocytes

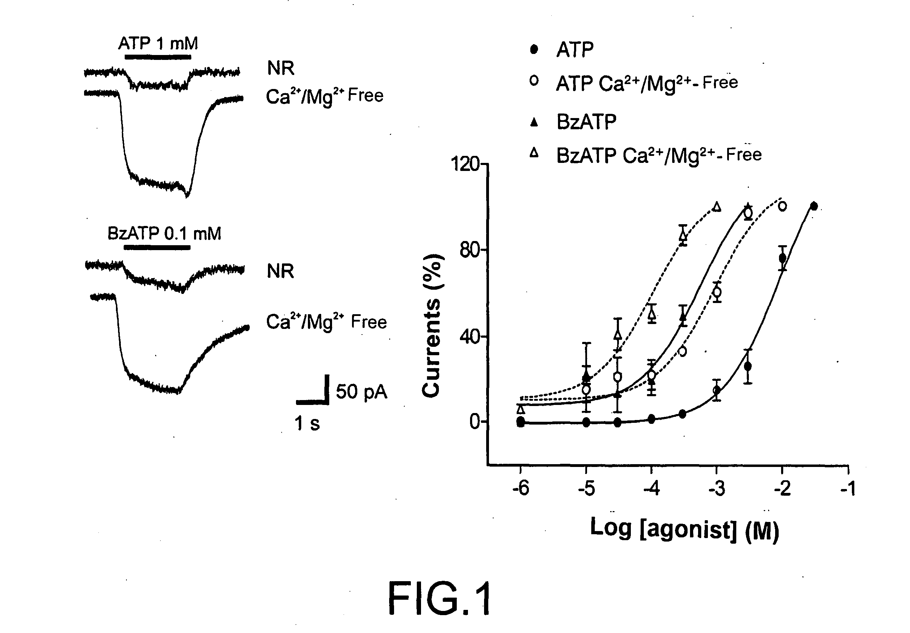

ATP (1 mM) induces an input current which does not desensitise in the majority of oligodendrocytes examined (77.3 % ± 7.9 ; n= 47; **Fig. 1a**). The ATP analogue, 2',3'-O-(4-benzoyl-4-benzoyl) (BzATP, 100 µM), which is a wide spectrum P2X agonist but with a higher affinity for the P2X7 receptor (Ralevic & Burnstock, 1998), also induced similar responses (**Fig. 1a**)**.** On the other hand, α,β-methylene-ATP (α,β-Me-ATP, 100 µM), a selective P2X1, P2X3 and P2X2/3 heteromers agonist, did not generate currents in oligodendrocytes. It was observed that the amplitude of currents generated by ATP and BzATP depends on the concentration of the corresponding agonist (**Fig. 1a**) (EC₅₀= 8.77 mM and 0.52 mM respectively). Likewise, it could be established that the absence of Mg²⁺ and Ca²⁺, which increase the concentration of ATP⁴⁻, the active form of the P2X receptors, increase the responses by 4-10 fold (**Fig. 1** **a**).

The PPADS wide spectrum antagonist (100 µM), for its part, completely blocked the currents induced by ATP (**Fig. 1**). For its part, oxidised ATP (o-ATP), a preferential antagonist of the P2X7 receptors, partially blocks the ATP currents. For its part, Cu²⁺ (1 mM) which is a selective inhibitor of the P2X7 receptors (Virginio et al, 1997), reduces ATP currents, while propofol (60µM), a potentiator of the P2X4 receptors, does not alter these currents. These results indicate that the P2X receptors present in oligodendrocytes have electrophysiological properties compatible with a predominance of the P2X7 sub-unit.

### The activation of P2X receptors increase the cytosol Ca²⁺ levels

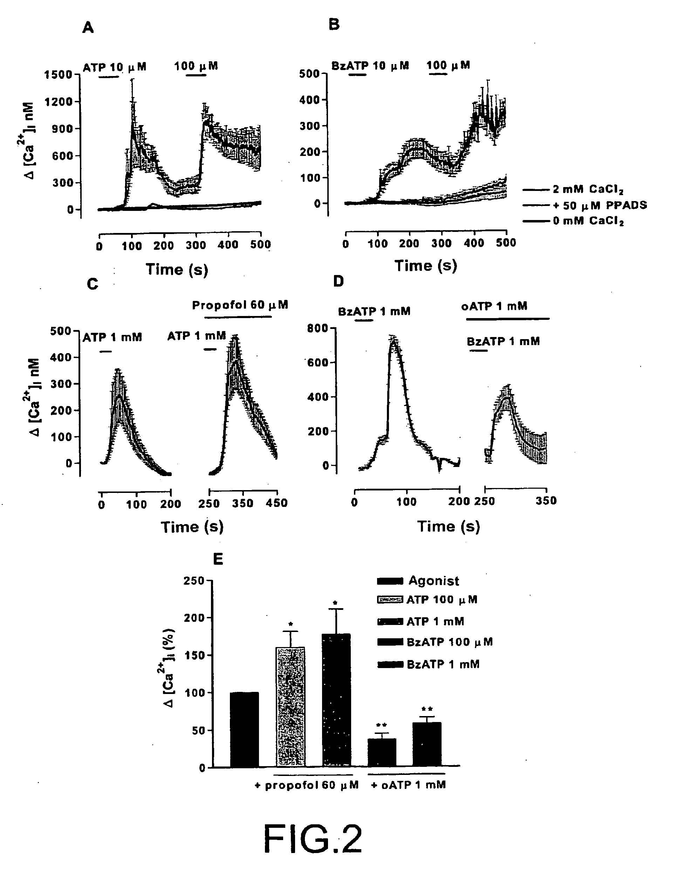

[Ca²⁺]ᵢ was monitored after applying ATP and BzATP with the objective of characterising the effects of the activation of P2X receptors on oligodendrocytes. These cells respond to ATP (10 µM) with a rapid increase in basal cytosol [Ca²⁺]ᵢ (250 ± 65 nM) to 1200 ± 468 nM (**Fig. 2a**). These responses are repressed in the presence of PPADS (50 µM) and with the absence of Ca²⁺ in the incubation solution. These results indicate that the [Ca²⁺]_{¡} increases are due to the entrance of Ca²⁺ across the plasma membrane and not due to the liberation of this from intracellular deposits.

Bz-ATP (0.01-1 mM) also activates the entrance of Ca²⁺ into oligodendrocytes in a dose dependent way (**Fig. 2b****, d**). This effect disappears in the absence of extracellular Ca²⁺ and is blocked by PPADS (**Fig. 2b****, d**). These results suggest that the P2X receptors which contain the P2X7 sub-unit are the principal mediators of the response to ATP. In agreement with this idea, the o-ATP P2X7 selective antagonist (1 mM) (Fernández et al, 2001), reduces the increase in [Ca²⁺] induced by Bz-ATP by 63 ± 8 (**Fig. 2d****, e**). For its part, propofol (60 µM), which triggers the responses mediated by P2X4 (Tomioka et al., 2000), promotes the increase of [Ca²⁺]ᵢ generated by 0.1 and 1 mM ATP in 60% ± 22 and 77% ± 34 respectively (**Fig. 2c****, d**). Therefore, the P2X native receptors which contain P2X4 also contribute to the entrance of calcium induced by ATP in oligodendrocytes.

### The activation of P2X receptors induce Ca²⁺ dependent oligodendroglial death

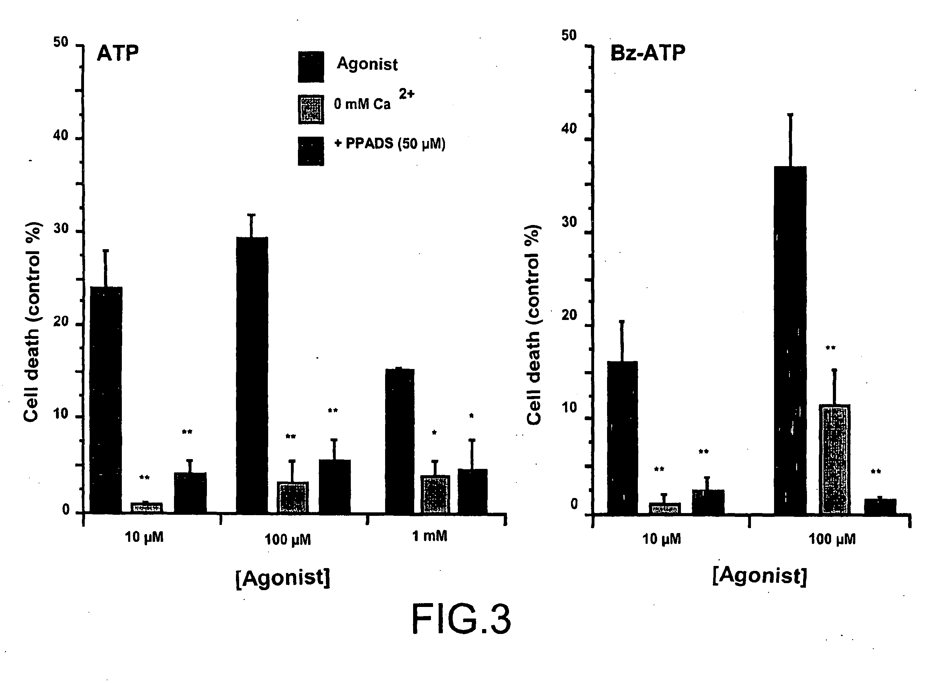

At all ATP concentrations (0.01-1 mM) tested, death was produced in 15-27% of oligodendrocytes which is inhibited in the presence of 50 µM PPADS and after removing Ca²⁺ from the culture medium (**Fig. 3a**). In the same way the Bz-ATP agonist caused a toxicity similar to ATP (**Fig. 3b**). Other purinergic agonists such as ATP-γ-S, which is a more stable analogue than ATP, and α,β,-meATP, are also toxic for the oligodendrocytes, which exclude the possibility that the metabolites of ATP might be the agents causing the toxicity after activating receptors different to the P2X ones. Overall, the toxicity tests showed that the oligodendrocytes are vulnerable to the activation of P2X receptors by ATP and its analogues.

### The oligodendrocytes express P2X receptors in oligodendrocytes in vitro and in situ

The analysis of the expression P2X receptors using immunohistochemistry with specific antibodies in cultures of differentiated oligodendrocytes (GalC⁺/MBP⁺) demonstrates that these cells mainly have the P2X2, P2X4 y P2X7 subunits (see Table 2).

**Table 2. Expression of P2X receptors in oligodendrocyte cultures.**

| **Subunit** | **P2x₁** | **P2x₂** | **P2x₃** | **P2x₄** | **P2x₅** | **P2x₆** | **P2x₇** |
|---|---|---|---|---|---|---|---|
| **Expression** | +/- | ++ | --- | ++ | +/- | +/- | ++ |

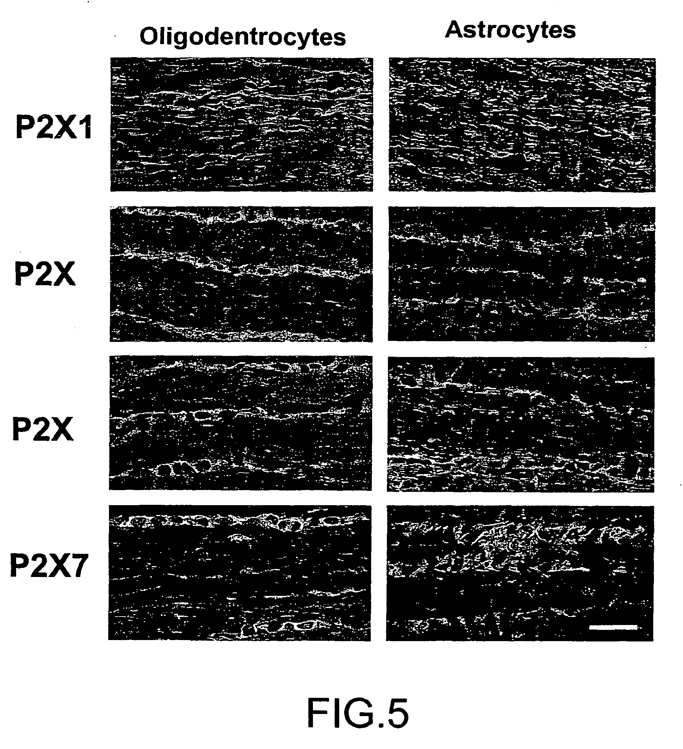

This expression profile is consistent with the electrophysiological properties and toxicity characteristics observed in these cultures. Also, the pattern of the subunits observed *in vitro* also corresponds with that observed *in situ* in optic nerve (Table 3) by means of double marking of the subunits and antibodies specific to the oligodendroglial and astroglial lineage (Fig.5).

**Table 3. Distribution of P2X receptors in oligodendrocytes in the optic nerve of the rat**

| **Subunit** | **P2X1** | **P2X2** | **P2X3** | **P2X4** | **P2X5** | **P2X6** | **P2X7** |
|---|---|---|---|---|---|---|---|
| **Distribution** | - | +++ | - | +++ | + | - | +++ |

These histochemical results were confirmed using Western blot (immunotransfer).

### ATP kills oligodendrocytes in situ

To determine if ATP is toxic to the oligodendrocytes in a preparation of nerve tissue without dissociating, entire optic nerves isolated from adult rats were perfused with artificial cerebrospinal fluid with ATP (100 µM) for 3 h. Under these conditions an increase of > 3 times the number of cells which showed nuclear condensation as compared to the control nerves perfused without ATP was produced (**Fig. 5**). The damaged cells are located in the longitudinal axis of the nerve and make up part of interfascicular oligodendrocyte rows. Stimulation with ATP in the presence of PPADS (10 µM) prevents the death of the oligodendrocytes.

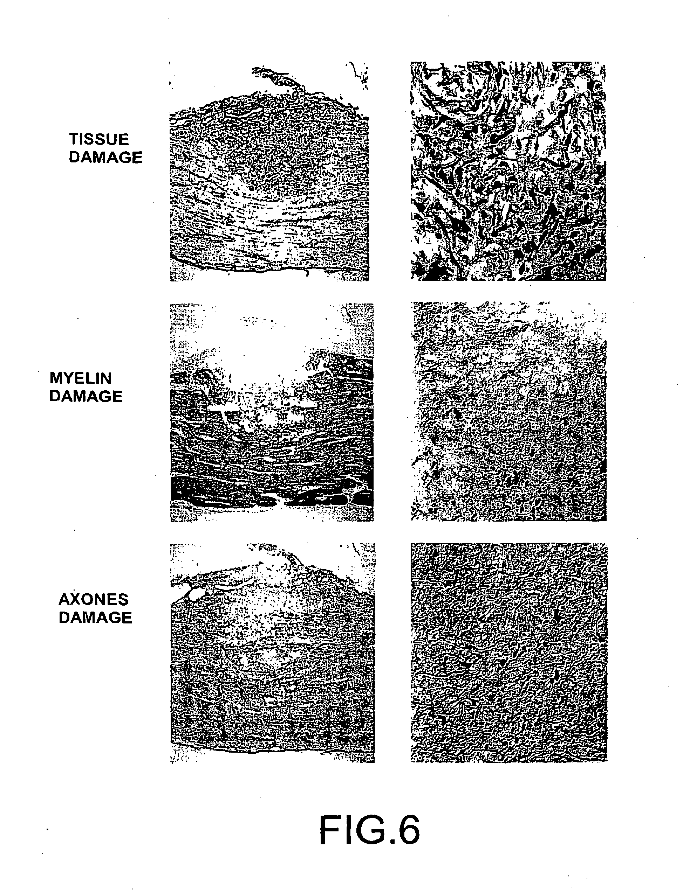

Next, the ATP-γ-S and BzATP agonists were infused over the optic nerve using osmotic pumps which released very small quantities of solute for 3 days. The histological examination of the nerves 7 days after starting the application showed tissue damage in an area restricted to the proximity of the cannula (**Fig. 6**). Also, this zone had intense gliosis, lack of myelin and axonal damage (**Fig. 6**). Overall these results indicate that the activation of P2X kills oligodendrocytes *in situ* and that the lesions *in vivo* share properties common to multiple sclerosis plaques.

### Blocking of P2X7 improves the motor symptoms of acute and chronic EAE

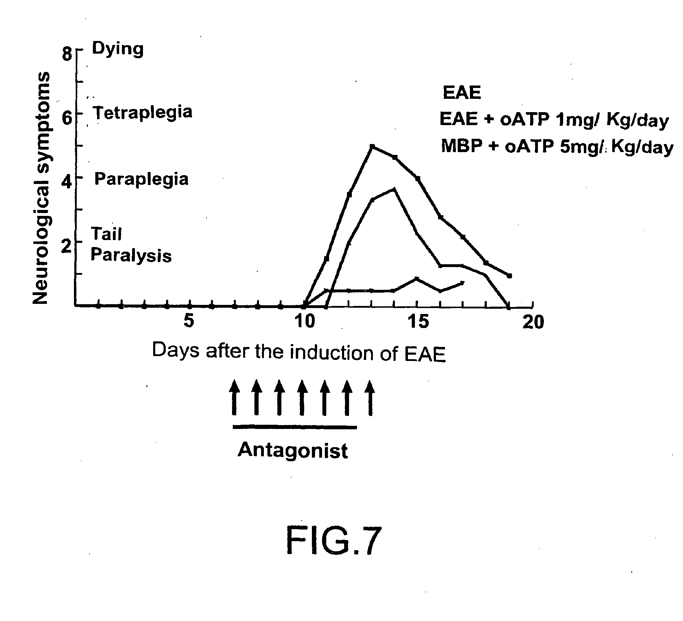

The effects of the wide spectrum antagonist PPADS and the more selective o-ATP in the triggering off and on the course of EAE induced by the immunisation of Lewis rats with basic myelin protein were investigated. The immunised rats showed signs of motor deficits around 10 days post-injection, and they reached a maximum at 14 days (**Fig. 7**). The treatment with PPADS (30 mg/kg, two times per day) from 7 to 14 days post-injection did not improve the symptoms or the course of the disease. On the other hand the application of o-ATP (1 and 5 mg/kg, every12 h) for the same period reduced or prevented the appearance of symptoms common to EAE (**Fig. 7**).

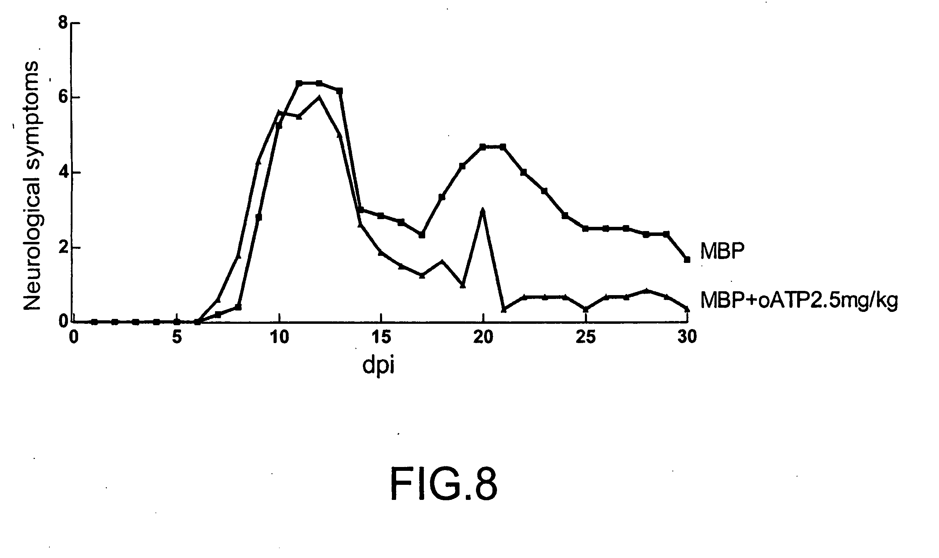

Later, the efficacy of o-ATP in improving the symptoms of EAE was evaluated in a chronic-recurrent-remittent model. For this, DA rats were immunised with syngeneic spinal cord, the appearance of severe neurological deficits being observed 7-9 days post-injection, and which reached their first peak around 11 days. Treatment with o-ATP (2.5 mg/kg, every 12 h), once the maximum intensity of the symptoms were established, reduced the symptoms and also eliminated those common to the chronic phase (**Fig. 8**).

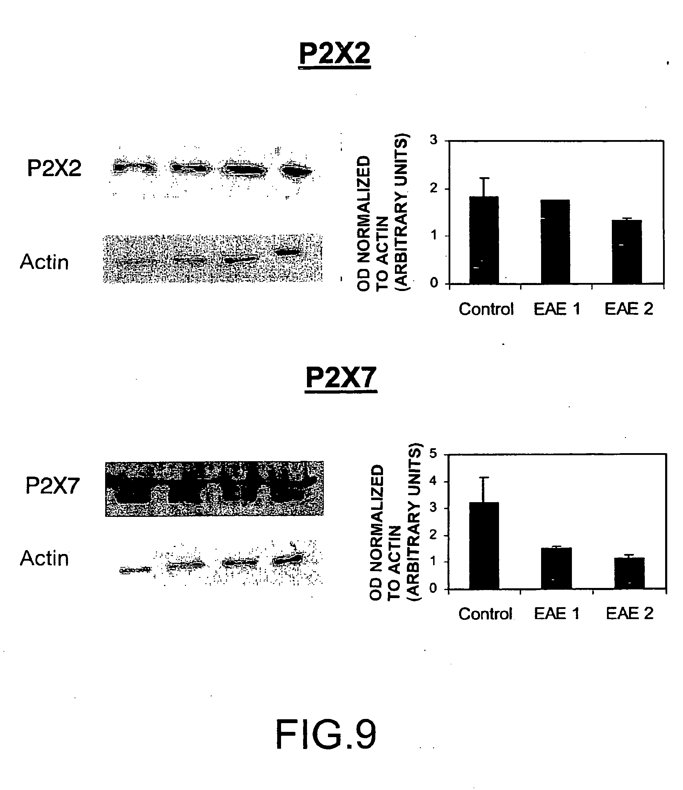

With the objective of understanding the mechanism of action by which o-ATP improves the prognosis of EAE, the levels of P2X7 receptors over which the preferred form of this drug acts on the lumbar-sacral spinal cord, the region most affected in this experimental disease, was evaluated using Western blot. We found that the levels of this subunit were reduced by half in animals subjected to EAE, and that these levels returned to those of the controls in those animals with EAE treated with o-ATP (**Fig. 9**). These results indicate that treatment with o-ATP protects the cells which express P2X7 from dying, and consequently, the oligodendrocytes, which are the main type of cells which express this subunit in the spinal cord.

### III- DISCUSSION

The results shown previously demonstrate for the first time that the oligodendrocytes have P2X receptors. Likewise, the electrophysiological, pharmacological and molecular properties of these receptors, as well as their increased permeability to calcium, are given in detail. This latter property results in that the oligodendrocytes may be vulnerable to intense and/or prolonged stimulus mediated by these receptors, as has been demonstrated with glutamergic receptors in this cell population (Matute et al, 2001, Trends Neurosci 24, 224-230). The vulnerability of the oligodendrocytes to the signals mediated by the P2X receptors is one of the causes of nervous tissue damage which underlies the experimental disease, EAE, a model of multiple sclerosis. Finally, blocking of the P2X7 receptors until triggering off the disease drastically reduces the neurological symptoms in acute EAE, and improves the outcome and prognosis in chronic EAE once the symptoms are established.

The invention described herein constitutes a means for the treatment of multiple sclerosis, a disease which lacks efficient treatments which slow down or check its progression. The routes of intervention which have resulted in the development of drugs in clinical trial phase or for use as drugs in the treatment of multiple sclerosis have mechanisms of action which regulate the functioning of the immune system. The fact that the blocking of the P2X7 may prevent the symptoms of acute EAE, a model of MS which mimics the inflammatory/autoimmune phase of the disease, indicates that these drugs can in fact be powerful immunomodulatory agents which may prevent the autoimmunity which triggers off MS and other diseases. Finally, the P2X7 receptor antagonists on being protector agents of the death of oligodendrocytes, the cell population which suffers most damage in MS, have great therapeutic potential in the neurodegenerative phase of this disease, a phase which is prolonged for decades and in which patients suffer a progressive deterioration which continues its course with motor and sensory disorders causing invalidity.

### REFERENCES

- Abbracchio, M.P. and Burnstock, G. (1998) Purinergic signalling: pathophysiological roles. Jpn. J. Pharmacol. 78: 113-145.
- Ansari KA, Rand A, Loch JA (1978) Biochemical and immunological studies with human optic and olfactory tracts J Neuropathol Exp Neurol 37:756-67
- Armstrong MA, Shah S, Hawkins SA, Bell AL, Roberts SD (1988) Reduction of monocyte 5'nucleotidase activity by gamma-interferon in multiple Ann Neurol 24:12-6
- Barnard, E.A., Simon, J. and Webb, T.E. (1997). Nucleotide receptors in the nervous system. An abundant component using diverse signal transduction mechanisms. Mol. Neurobiol. 15: 103-129.
- Braun, N., Zhu, Y., Krieglstein, J., Clumsee, C. and Zimmermann, H. (1998). Upregulation of the enzyme chain hydrolysing extracellular ATP after transient forebrain ischemia in the rat. J. Neurosci. 18: 4891-4900.
- Ciccarelli, R. Ballerini, P., Sabatino, G., Rathbone, M.P., D'Onofrio, M.. Caciagli, F. and lorio, P. (2001). Involvement of astrocytes in purine-mediated reparative processes in the brain. Int. J. Dev. Neurosci. 19: 395-414.
- Dunn, P.M., Zhong, Y. and Burnstock, G. (2001). P2X receptors in peripheral neurons. Prog. Neurobiol. 65:107-134.
- Fields, R.D. and Stevens, B. (2000). ATP: an extracellular signalling molecule between neurons and glia. Trends Neurosci. 23: 625-633.
- Franke, H., Grosche, J., Schädlich, H., Krügel, U., Allgaier, C. and Illes, P. (2001 a). P2X receptor expression on astrocytes in the nucleus accumbens of rats. Neuroscience 108: 421-429.
- Franke, H., Krugel, U., Schmidt, R., Grosche, J., Reichenback, A. and Illes, P. (2001 b). P2 receptor-types involved in astrogliosis in vivo. Brit. J. Pharmacol. 134: 1180-1189.
- Honda, S. and Kohsaka, S. (2001). Regulation of microglial cell function by ATP. Nihon Shinke 21: 89-93.
- James, G. and Butt, A.M. (2001). Changes in P2Y and P2X purinoceptors in reactive glia following axonal degeneration in the rat optic nerve. Neurosci. Lett. 212: 33-36.
- Matute C, Alberdi E, Domercq M, Pérez-Cerdá F, Pérez-Samartín A and Sanchez-Gomez MV (2001) The link between excitotoxicity and demyelinating diseases. Trends Neurosci. 24, 224-230.
- Matute C, Alberdi E, Ibarretxe G and Sánchez-Gómez MV (2002) Excitotoxicity in glial cells. Eur. J. Pharmacol 447:239-246.
- Queiroz, G., Gebicke-Haerter, P.J., Schobert, A., Starke, K. and von Kugelgen, I. (1997). Release of ATP from cultured rat astrocytes elicited by glutamate receptor activation. Neuroscience 78: 1203-1208.
- Ralevic, V. and Burnstock, G. (1998) Receptors for purines and pyrimidines. Pharmacol. Rev. 50: 413-492.
- Rathbone, M.P., Meddlemiss, P.J., Gysbers, J.W., Andrew, C., Herman, M.A., Reed, J.K., Ciccarelli, R. Di lorio, P. and Caciagli, F. (1999). Trophic effects of purines in neurons and glia. Prog. Neurobiol. 59: 663-690.
- Stevens, B, Porta, S., Haak, L.L., Gallo, V. and Fields, R.D. (2002) Adenosine: a neuron-glial transmitter promoting myelination in the CNS in response to action potentials. Neuron 36: 855-868.
- Zamvil, S.S. and Steinman L. (2003) Diverse targets for intervention during inflammatory and neurodegenerative phases of multiple sclerosis. Neuron 38, 685-688.

## Claims

1. Use of the antagonist of P2X7 purinergic receptors o-ATP or BBG in the preparation of a drug for the treatment of the neurodegenerative phase of multiple sclerosis, in mammals, including man.

## Patentansprüche

1. Verwendung des Antagonisten der P2X7 purinergen Rezeptoren o-ATP oder BBG zur Herstellung eines Medikaments zur Behandlung der neurodegenerativen Phase von Multipler Sklerose in Säugern, einschliesslich Menschen.

## Revendications

1. Utilisation de l'antagoniste o-ATP ou BBG des récepteurs purinergiques P2X7 pour la préparation d'un médicament pour le traitement de la phase neurodégénérative des scléroses multiples, chez l'animal dont l'Homme.
